# EUROPEAN PATENT APPLICATION

(11) **EP 3 578 642 A1**
(43) Date of publication of application: **11.12.2019**
(21) Application number: 18747649.4
(22) Date of filing: 30.01.2018
(51) Int. Cl.: C12N 5/0784, A61K 35/15

(54) **PLASMACYTOID DENDRITIC CELLS HAVING IMMUNE TOLERANCE, AND METHOD FOR PRODUCING SAME**

(30) Priority: 31.01.2017 KR 20170014018
(71) Applicant: Quratis Inc., Seoul 08375 (KR)
(72) Inventor: SHIN, Sung Jae, Seoul 03722 (KR); KIM, Woo Sik, Seoul 03722 (KR); KIM, Hong Min, Seoul 03722 (KR)
(74) Representative: Cabinet Becker et Associés
(86) International application number: PCT/KR2018/001299
(87) International publication number: WO 2018/143652

(57) **Abstract**

The present invention relates to a production method of tolerogenic plasmacytoid dendritic cells, tolerogenic plasmacytoid dendritic cells produced by the method, and, cell therapeutic agents including the same. In the method, the plasmacytoid dendritic cells with immune tolerance may be induced from the immature dendritic cells at a high yield using a simple and easy process, thereby enabling a stable supply of large amounts of tolerogenic plasmacytoid dendritic cells.

## Description

### CROSS-REFERENCE TO RELATED APPLICATIONS

This application is based on and claims priority from Korean Patent Application No. 10-2017-0014018, filed on January 31, 2017, with the Korean Intellectual Property Office, the disclosure of which is incorporated herein in its entirety by reference.

### TECHNICAL FIELD

The present disclosure relates to a production method of tolerogenic plasmacytoid dendritic cells, tolerogenic plasmacytoid dendritic cells produced by the method, and cell therapeutic agents including the same.

### BACKGROUND

In the immune system, the immune response and the immune tolerance reaction must be coordinated and balanced with each other. When an over-immune response occurs, for example, rheumatoid arthritis, autoimmune diseases of type 1 diabetes, sepsis and hypersensitivity allergic diseases may occur. They have a great impact on human survival and quality of life.

Previously, it was thought that dendritic cells act as an important role only in inducing immune responses. Recently, it has become known that the dendritic cells greatly contribute to the immune tolerance reaction. Thus, researches on the dendritic cells related to the immune tolerance reaction have become active.

The dendritic cells play a pivotal role not only in innate immunity but also in controlling acquired immunity, which is selectively induced by acquired causation. Specifically, the dendritic cell is a type of an antigen presenting cell that performs a function of presenting information about an antigen invaded from the outside to a T cell.

Further, the dendritic cells play a bridge between innate immunity and acquired immunity. The dendritic cells are capable of priming naive T cells among immune cells. The dendritic cells are present in a form of branches in an intercellular space of each of various tissues including lymphoid tissue. Further, the dendritic cells are finally differentiated cells, and are present in 1% or less of total immune cells in the body. However, the dendritic cells are capable of inducing lymphocyte activity much more strongly than monocyte or macrophage cells. The dendritic cells have an immature form originating from the bone marrow and may be transferred to all the organs through the bloodstream. Therefore, the dendritic cells may play an important role in the T cell activation by collecting the antigen around each tissue and moving to the lymphatic organs and then transferring the antigen to T lymphocytes.

Thus, recently, the dendritic cells have been used as immunizing vaccines or immunotherapeutic agents. The immunization vaccines and immunotherapeutic agents are all known to be safe without side effects and their use is increasing for treating various diseases.

The dendritic cells are composed of various subpopulations depending on their origin, phenotype, and function. In particular, plasmacytoid dendritic cells (pDC) may produce type I IFN at high levels in response to stimuli derived from various pathogens. The plasmacytoid dendritic cells are distinguished from conventional dendritic cells (cDC) based on the surface phenotypes. The pDC expresses B220 and expresses CD11c at a low level. However, the cDC expresses both CD11c and CD11b at high levels and does not express B220. Both the pDC and cDC are obtained from a process by which the immature dendritic cells are mature so that the pDC and cDC can act as effective stimulants for a harmonious immune response.

As noted, pDC is known to play an important role in defense against virus infection by activating immune cells via secretion of large amounts of type I IFNs upon infection by DNA viruses or single-stranded RNA viruses. In particular, pDC strongly activates mature dendritic cells (mDCs) capable of presenting antigens, thereby helping to amplify antiviral responses by T cells. However, studies on the differentiation or role of pDC in the body have been not vigorous compared to other dendritic cells.

Recently, the use of the dendritic cells for the treatment of autoimmune diseases has become prominent. The autoimmune disease is a chronic disease that is estimated to have billions of patients worldwide. There is an urgent need for a production technology of tolerogenic dendritic cells that maintain or enhance immunity tolerance consistently by controlling the expression of specific genes responsible for the immunity of the dendritic cells or discovering immune tolerance inducing substances. In particular, although there are many studies that pDCs play an important role in various autoimmune or infectious diseases, a systematic and standardized differentiation method for producing tolerogenic pDCs is unknown.

### SUMMARY

The inventors of the present disclosure discovered that treatment of a toll-like receptor agonist (TLR agonist) to the immature dendritic cells to initiate the differentiation of the immature dendritic cells or treatment of the toll-like receptor agonist during the differentiation of the immature dendritic cells results in inducing tolerogenic plasmacytoid dendritic cells. In this way, the present disclosure has been achieved.

A purpose of the present disclosure is to provide a method for mass production of tolerogenic plasmacytoid dendritic cells from the immature dendritic cells.

Another purpose of the present disclosure is to provide plasmacytoid dendritic cells that are produced by the method and consistently tolerogenic.

Still another purpose of the present disclosure is to provide a cell therapeutic agent capable of inducing differentiation into regulatory T cells and inhibiting the activity of effector T cells among the immune cells using the tolerogenic plasmacytoid dendritic cells.

Other purposes and advantages of the present disclosure will become more apparent from the following detailed description of the invention, claims and drawings.

An exemplary embodiment of the present disclosure provides a production method of tolerogenic plasmacytoid dendritic cells (pDC), the method including a step of treating the immature dendritic cells with toll-like receptor agonists.

Further, another exemplary embodiment of the present disclosure provides tolerogenic plasmacytoid dendritic cells induced by treating the immature dendritic cells with toll-like receptor agonists.

Furthermore, still another exemplary embodiment of the present disclosure provides a cell therapeutic agent that includes the tolerogenic plasmacytoid dendritic cells.

According to the exemplary embodiments of the present disclosure, the tolerogenic plasmacytoid dendritic cells may be induced from the immature dendritic cells at a high yield using a simple and easy process, thus enabling a stable supply of large numbers of the tolerogenic plasmacytoid dendritic cells.

Further, according to the exemplary embodiments of the present disclosure, the tolerogenic plasmacytoid dendritic cells obtained as described above induce expression of anti-inflammatory cytokines and suppress expression of inflammatory cytokines and promote differentiation into regulatory T cells, thereby to effectively prevent or treat autoimmune diseases, hypersensitivity immune diseases or allergic diseases.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

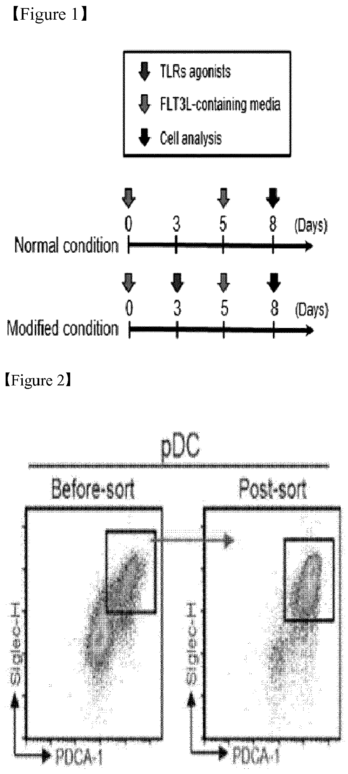

FIG. 1 shows a schematic design diagram of a treatment method of the immature dendritic cells using a differentiation-inducing factor (Flt3L-containing media) and toll-like receptor agonists (TLRs agonists) according to one embodiment of the present disclosure.
FIG. 2 shows separation of plasmacytoid dendritic cells (TLRs-pDC) as differentiated in an induced manner after treatment using the toll-like receptor agonist (Pam3) according to one embodiment of the present disclosure in Example 1.

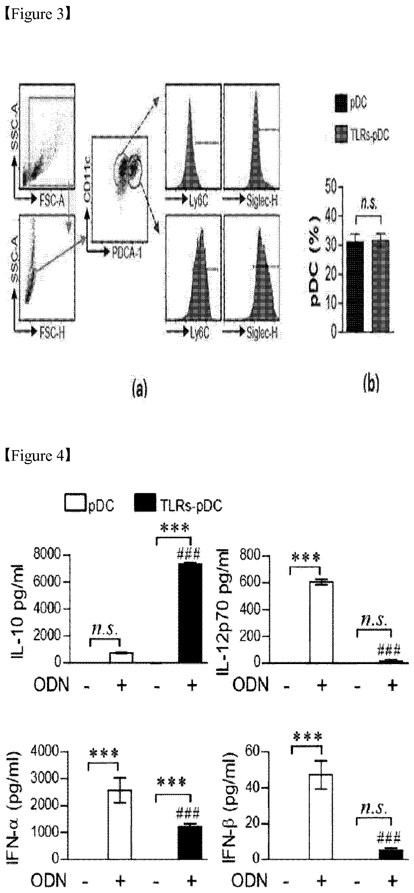

FIG. 3A shows surface expression molecules specifically induced to the plasmacytoid dendritic cells as differentiated in an induced manner after treatment using the toll-like receptor agonist (Pam3) according to one embodiment of the present disclosure in Example 1.
FIG. 3B shows a ratio of the number of plasmacytoid dendritic cells as differentiated in an induced manner after treatment using the toll-like receptor agonist (Pam3) according to one embodiment of the present disclosure, compared to the number of the immature dendritic cells in Example 1.
FIG. 4 is a graph showing comparison results between expression patterns of cytokine (IFN-α, IFN-β, IL-12p70, and IL-10) from the plasmacytoid dendritic cells (TLRs-pDC) as differentiated in an induced manner after treatment using the toll-like receptor agonist (Pam3) according to one embodiment of the present disclosure and the conventional plasmacytoid dendritic cells (pDC) in Example 2.

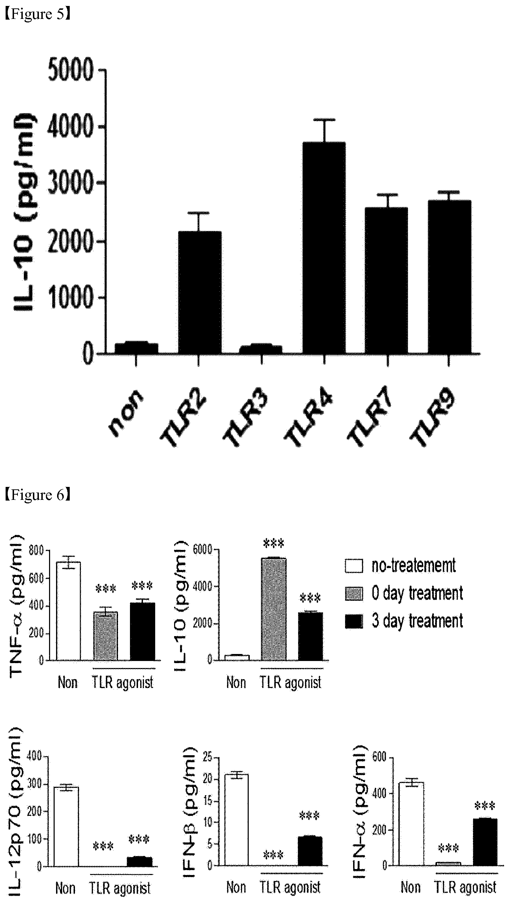

FIG. 5 is a graph showing comparison results between expression patterns of cytokine (IL-10) from the plasmacytoid dendritic cells as differentiated in an induced manner after treatment using various toll-like receptor agonists according to one embodiment of the present disclosure and the conventional plasmacytoid dendritic cells (non) in Example 3.
FIG. 6 is a graph showing comparison results between expression patterns of cytokine (IFN-α, IFN-β, TNF-α, IL-12p70, and IL-10) based on treatment timing of the immature dendritic cells using the toll-like receptor agonist (Pam3) according to one embodiment of the present disclosure in Example 4.

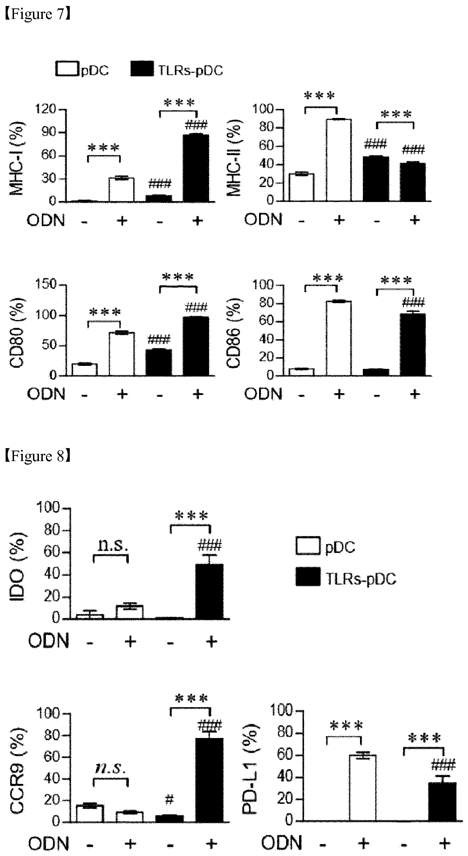

FIG. 7 is a graph showing comparison results between expression patterns of MHC complex molecule, CD80, and CD86 from the plasmacytoid dendritic cells (TLRs-pDC) as differentiated in an induced manner after treatment using the toll-like receptor agonist (Pam3) according to one embodiment of the present disclosure and the conventional plasmacytoid dendritic cells (pDC) in Example 5.
FIG. 8 is a graph showing comparison results between expression patterns of IDO, CCR9, and PD-L1 from the plasmacytoid dendritic cells (TLRs-pDC) as differentiated in an induced manner after treatment using the toll-like receptor agonist (Pam3) according to one embodiment of the present disclosure and the conventional plasmacytoid dendritic cells (pDC) in Example 6.

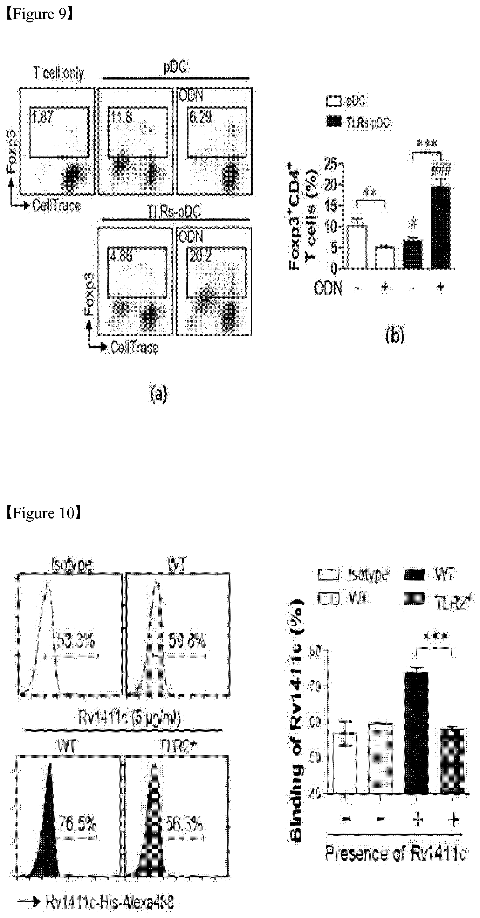

FIG. 9A shows measurement results using a flow cytometer LSRFortessa x-20 of whether proliferation into regulatory T cells is achieved by treatment using the plasmacytoid dendritic cells (TLRs-pDC) as differentiated in an induced manner after treatment using the toll-like receptor agonist (Pam3) according to one embodiment of the present disclosure or using the conventional plasmacytoid dendritic cells (pDC) in Example 7 regarding T cells.
FIG. 9B shows measurement results of a proliferation rate of regulatory T cells by treatment using the plasmacytoid dendritic cells (TLRs-pDC) as differentiated in an induced manner after treatment using the toll-like receptor agonist (Pam3) according to one embodiment of the present disclosure or using the conventional plasmacytoid dendritic cells (pDC) in Example 7 regarding T cells.
FIG. 10 shows the bindings between wild-type dendritic cells (WT) and dendritic cells (TLR2 -/-) isolated from TLR2 knockout mice and Rv1411c protein in Example 8.

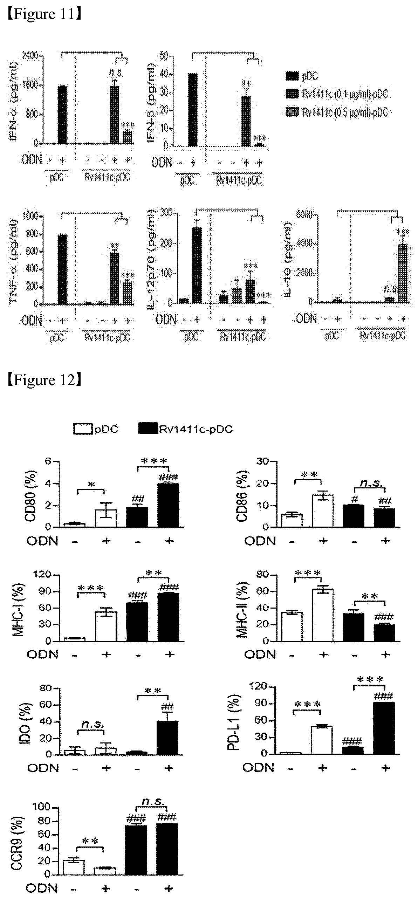

FIG. 11 is a graph showing comparison results between expression patterns of cytokine (IFN-α, IFN-β, TNF-α, IL-12p70, IL-10) from plasmacytoid dendritic cells (Rv1411c(0.1µg/ml)-pDC, Rv1411c (0.5µg/ml)-pDC) as differentiated in an induced manner after treatment using a toll-like receptor agonist (Rv1411c protein(0..1 µg/ml, 0.5 µg/ml)) according to one embodiment of the present disclosure and from the conventional plasmacytoid dendritic cells (pDC) in Example 9.
FIG. 12 is a graph showing comparison results between expression patterns of MHC complex molecule, CD80, CD86, and immune tolerance-inducing molecule (IDO, CCR9 and PD-L1) from the plasmacytoid dendritic cells (Rv1411c-pDC) as differentiated in an induced manner after treatment using the toll-like receptor agonist (Rv1411c protein) according to one embodiment of the present disclosure and the conventional plasmacytoid dendritic cells (pDC) in Example 10.

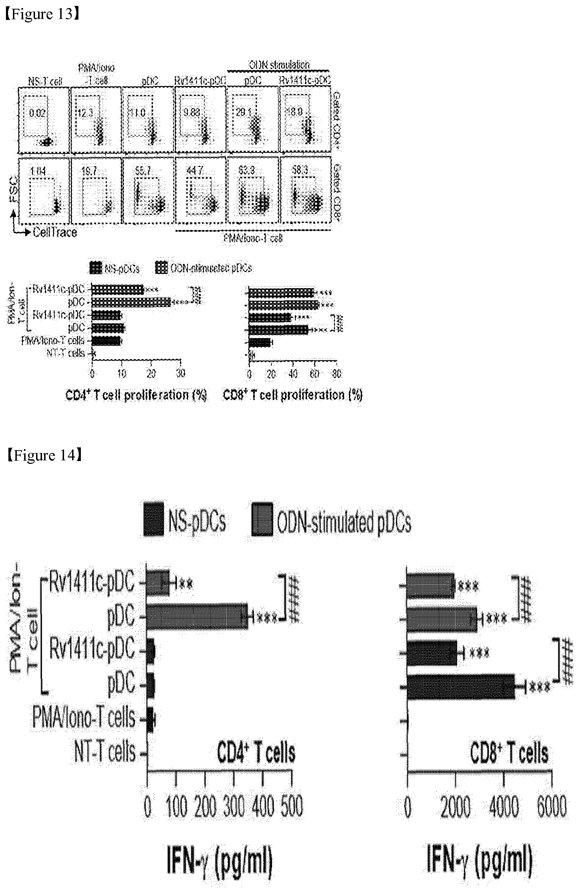

FIG. 13 shows comparison results of T cell proliferation as obtained by mixing and culturing T cells and the plasmacytoid dendritic cells (Rv1411c-pDC) as differentiated in an induced manner after treatment using the toll-like receptor agonist (Rv1411c protein) according to one embodiment of the present disclosure and T cell proliferation as obtained by mixing and culturing T cells and the conventional plasmacytoid dendritic cells (pDC) in Example 11.
FIG. 14 shows comparison results of a secretion pattern of IFN-gamma as obtained by mixing and culturing T cells and the plasmacytoid dendritic cells (Rv1411c-pDC) as differentiated in an induced manner after treatment using the toll-like receptor agonist (Rv1411c protein) according to one embodiment of the present disclosure and a secretion pattern of IFN-gamma as obtained by mixing and culturing T cells and the conventional plasmacytoid dendritic cells (pDC) in Example 11.

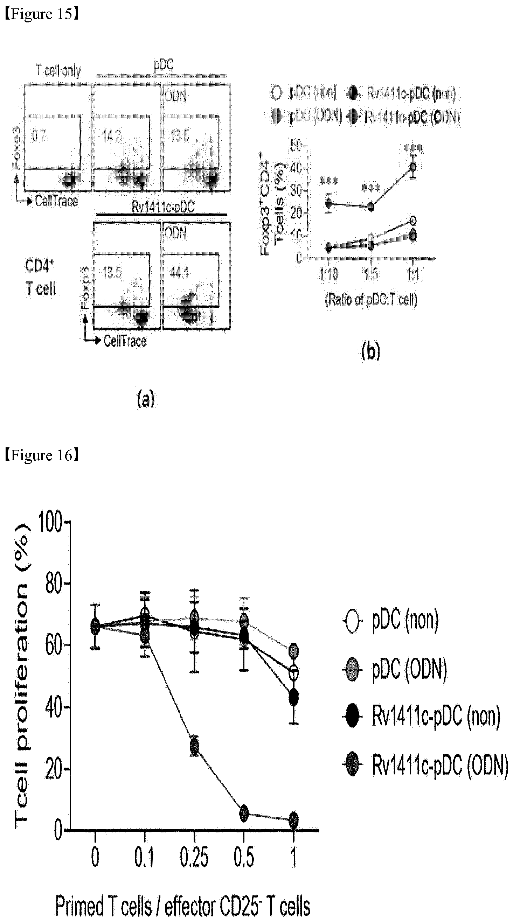

FIG. 15A shows measurement results using a flow cytometer LSRFortessa x-20 of whether proliferation into regulatory T cells is achieved by treatment using the plasmacytoid dendritic cells (Rv1411c-pDC) as differentiated in an induced manner after treatment using the toll-like receptor agonist (Rv1411c protein) according to one embodiment of the present disclosure or using the conventional plasmacytoid dendritic cells (pDC) in Example 12 regarding T cells.
FIG. 15B shows measurement results of a proliferation rate of regulatory T cells by treatment using the plasmacytoid dendritic cells (Rv1411c-pDC) as differentiated in an induced manner after treatment using the toll-like receptor agonist (Rv1411c protein) according to one embodiment of the present disclosure or using the conventional plasmacytoid dendritic cells (pDC) in Example 12 regarding T cells.
FIG. 16 is a graph showing the results of measurement of changes in T cell proliferation ability based on a ratio of primed T cells to CD25-effector T cells, as obtained after culturing, together with CD4 + and CD25-effector T cells, T cells as differentiated by the plasmacytoid dendritic cells (Rv1411c-pDC) as differentiated in an induced manner after treatment using the toll-like receptor agonist (Rv1411c protein) according to one embodiment of the present disclosure in Example 13.

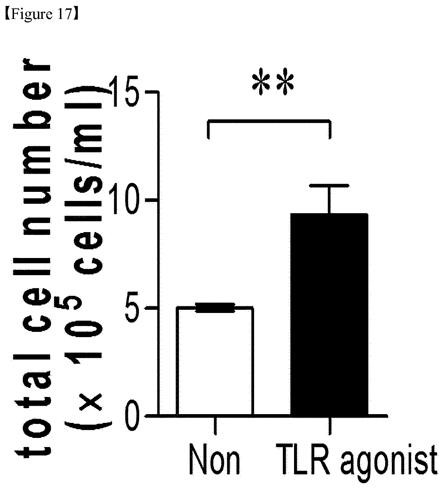

FIG. 17 a graph showing comparison results between the number of plasmacytoid dendritic cells (TLR agonist) induced after treatment with toll-like receptor agonist (RYv1411c protein and Pam3) according to one embodiment of the present disclosure and the number of the plasmacytoid dendritic cells induced after non-treatment (Non) using the toll-like receptor agonist in Example 14.

In FIG. 1 to FIG. 17, * means P < 0.05, ** means P < 0.01 and *** means P <0 .005.

### DETAILED DESCRIPTION

In the following detailed description, reference is made to the accompanying drawing, which forms a part hereof. The illustrative embodiments described in the detailed description, drawing, and claims are not meant to be limiting. Other embodiments may be utilized, and other changes may be made, without departing from the spirit or scope of the subject matter presented herein.

The present disclosure relates to the production method of tolerogenic plasmacytoid dendritic cells, the method including the step of treating the immature dendritic cells with toll-like receptor agonists.

As used herein, the term, "dendritic cells (DCs)" refer to a professional antigen presenting cell for absorbing the antigen into the cell and presenting various antigenic samples to the T cell together with MHC (major histocompatibility complex) class I complex or MHC class II complex. Further, the dendritic cells may include both immunogenic and tolerogenic antigen presenting cells, and may be classified into the immature dendritic cells ("imDC"), semimature dendritic cells ("smDC") and mature dendritic cells ("mDC") according to their maturity.

As used herein, the term, "immature dendritic cells" are found in the early mature stages of the dendritic cells. The immature dendritic cell does not express CD14 as the surface phenotype of the monocyte cell. The immature dendritic cell may express one of the co-stimulatory molecules CD40, CD54, CD80, CD86 and CD274 at a lower level than the mature dendritic cells.

As used herein, the term, "semimature dendritic cells" refer to dendritic cells that lose some of the properties of the immature dendritic cells, and have some characteristics of the phenotype of mature dendritic cells. This semimature dendritic cell may mean a dendritic cell that exhibits partially or incompletely mature form and phenotypic characteristics.

As used herein, the term, "mature dendritic cells" refer to cells into which the immature dendritic cells are matured. The mature dendritic cells express high levels of MHC class II, CD40, CD54, CD80, CD86 and CD274 as well as DC-LAMP, and release anti-inflammatory cytokines. The mature dendritic cells may be characterized by having the ability to cause an increase in proliferation of primitive allogeneic T cells and syngeneic T cells and/or an increased production of dendritic cells cytokines in a mixed lymphocyte reaction. The mature dendritic cells typically express high levels of CCR7 and CXCR4.

However, in accordance with the present disclosure, the dendritic cells to be treated with the toll-like receptor agonist are preferably the immature dendritic cells that have not undergone differentiation. In this connection, the immature dendritic cells may include naive dendritic cells and may be isolated and obtained from the mammalian bone marrow and the like.

Further, in accordance with the present disclosure, the tolerogenic dendritic cells differentiated from the immature dendritic cells by treating the immature dendritic cells using the toll-like receptor agonist may be tolerogenic plasmacytoid dendritic cells (pDC).

As used herein, the term, "plasmacytoid dendritic cells" are a subset of dendritic cells. The plasmacytoid dendritic cell was first known by histologically finding the shape of a plasma cell in a human lymph node by Dr. Lennert and Dr. Remmele in 1958. It is known that the plasmacytoid dendritic cells do not express B cell-specific marker (immunoglobulin). Thus, the plasmacytoid dendritic cells are named as plasmacytoid T cells. It is known that the plasmacytoid dendritic cells express myeloid lineage antigen and MHC class II while not expressing CD3 which is a common marker of the T cell. Thus, the plasmacytoid dendritic cells are named as plasmacytoid monocytes. Thereafter, it is confirmed that the plasmacytoid dendritic cells have the ability to induce an allogeneic mixed lymphocyte reaction (MLR), which is an intrinsic property of dendritic cells. Thus, the plasmacytoid dendritic cells are called plasmacytoid dendritic cells, simply, as 'pDC'.

As used herein, the term "tolerogenic" refers not only to a state that the cell does not exhibit an immune response to a specific antigen, but also to a state that the cell inhibits the immune response. Therefore, in accordance with the present disclosure, the "tolerogenic plasmacytoid dendritic cells" promote secretion of anti-inflammatory cytokines such as IL-10, and inhibit the secretion of inflammatory cytokines such as IL-12p70 and TNF-α. Recently, the tolerogenic plasmacytoid dendritic cells express indoleamine-2,3 dioxygenase (IDO) known as immune tolerance-inducing molecules, and CCR9 as a surface molecule of the immune tolerance-inducing cells at high levels, thereby to induce differentiation of regulatory T cells and to inhibit the activity of effector T cells.

In accordance with the present disclosure, the tolerogenic plasmacytoid dendritic cells may be produced stably and in large quantities by treating the immature dendritic cells using the toll-like receptor agonist before differentiation initiation or during the differentiation of the immature dendritic cells. In this connection, the 'before differentiation initiation' may include the time before the differentiation-inducing factor is applied to the immature dendritic cells. Further, the duration 'during differentiation' refers to the duration from a time when the differentiation-inducing factor is applied to the immature dendritic cells to a time before the differentiation is completed by the differentiation-inducing factor. Preferably, the duration 'during differentiation' may include duration from the time of the treating of the differentiation-inducing factor to 7 days after the treatment. However, the present disclosure is not limited thereto.

In accordance with the present disclosure, the application timing of the toll-like receptor agonist is not limited to a specific time point as long as the toll-like receptor agonist is applied prior to or during differentiation of the immature dendritic cells as described above. In accordance with the present disclosure, when the toll-like receptor agonist is applied thereto during the differentiation of the immature dendritic cells, the toll-like receptor agonist may be applied thereto within 7 days (168 hours), 5 days (120 hours) or 3 days (72 hours) from the differentiation initiation. However, the present disclosure is not limited thereto. Further, in accordance with the present disclosure, when the toll-like receptor agonist is applied thereto prior to the initiation of differentiation of the immature dendritic cells, the differentiation-inducing factors may be used to initiate the differentiation of the immature dendritic cells within 36 hours, preferably 24 hours, from the application of the toll-like receptor agonist. However, the present disclosure is not limited thereto.

Further, in accordance with the present disclosure, the toll-like receptor agonist may be applied once or more. The specific application times are not particularly limited. In one example, when applying the toll-like receptor agonist thereto during the differentiation of the immature dendritic cells, the toll-like receptor agonist may be applied thereto at least once within 7 days, 5 days or 3 days from the time of differentiation initiation of the immature dendritic cells. Further, when treating the toll-like receptor agonist prior to the initiation of differentiation of the immature dendritic cells, the differentiation of the immature dendritic cells may be initiated within 36 or 24 hours from any one treatment time point after the treatment of the immature dendritic cells with the toll-like receptor agonist at least once, and then, one or more additional treatments may optionally be carried out during the differentiation.

In this connection, the "differentiation initiation" refers to the addition of the differentiation-inducing factor to a culture medium of the immature dendritic cells, or to the inoculation of the immature dendritic cells into a medium containing the differentiation-inducing factors. However, the differentiation initiation is not particularly limited as long as the differentiation initiation may induce the differentiation of the immature dendritic cells using the differentiation-inducing factor.

As used herein, the term, "toll-like receptor agonist" may refer to a conserved molecular substance derived from a pathogen and may be pathogen-associated molecular patterns (PAMPs). In this connection, the pathogen may be gram-positive bacteria, gram-negative bacteria, fungi or viruses. Further, the toll-like receptor agonist may be endogenous molecules released from damaged or dead cells and may be damage-associated molecular patterns (DAMPs). DAMPs or PAMPs initiate an immune response via the TLR signal and collect adapter molecules within cytoplasm of the cell to deliver the signal. The toll-like receptor agonist may be fragments, variants, analogs, homologs or derivatives of PAMPs or DAMPs that bind to the toll-like receptors, and induce TLR-mediated activation, such as activation of NF-κB. The fragments, variants, analogs, homologs or derivatives as the toll-like receptor agonist are at least 30 to 99% identical to the amino acids of the TLR agonist and induce toll-like receptor-mediated activation.

The type of toll-like receptor agonist applied to the immature dendritic cells in accordance with the present disclosure is not particularly limited but, preferably, is a PAMPs ligand. More preferably, the type of toll-like receptor agonist applied to the immature dendritic cells is capable of inducing the differentiation of the tolerogenic plasmacytoid dendritic cells from the immature dendritic cells via MyD88 (myeloid differentiation primary response gene 88) signal.

Further, in accordance with the present disclosure, the toll-like receptor agonist includes at least one selected from the group consisting of a TLR2 agonist, a TLR4 agonist, a TLR5 agonist, a TLR7 agonist, a TLR8 agonist, a TLR9 agonist, a TLR11 agonist, a TLR12 agonist and a TLR13 agonist. More specifically, one or more of the ligands shown in Table 1 below may be included in the toll-like receptor agonist. However, the present disclosure is not particularly limited thereto. As long as any ligand can be bound to the toll-like receptor of the dendritic cell and may encounter the MyD88 signal, any ligand may be used without limitation.

Further, in accordance with the present disclosure, when the TLR2 agonist is used as the toll-like receptor agonist, one or more of the TLR1 and TLR6 agonists acting as the co-receptor for the TLR2 agonist may be further included in the toll-like receptor agonist. In this connection, specific examples of the TLR1 agonist and TLR6 agonist may be the ligands shown in Table 1 below. The ligands as the TLR1 agonist and TLR6 agonist may not be particularly limited as long as the ligands are capable of acting as a co-receptor for the TLR2 agonist.

**[Table 1]**

| Ligand | PAMPs(Pathogen Associated Ligands) | Ligand natural host | Synthetic ligand |
|---|---|---|---|
| TLR1 | Multiple triacyl lipopeptides | Bacteria | Pam3Cys-* |
| TLR2 | Multiple glycolipids | Bacteria | CFA |
| | Multiple lipopeptides | Bacteria | Pam3, MALP2-** |
| | Multiple lipoproteins | Bacteria | Pam2Cys** |
| | Ipoteichoic acid | Gram-positive bacteria | FSL-1 |
| | HSP 70, (other heat shock proteins) | Host cells | Hib-OMPC |
| | Zymosan (Beta- glucan) | Fungi | |
| | Protein | Mycobacteriu m tuberculosis | Rv1411c protein, ESAT-6, PE/PPE Protein, Rv0577 |
| TLR3 | Double-stranded RNA | Virus | Poly (I : C); Low molecular weight or high molecular weight Poly (A : U) |
| TLR4 | Lipopolysaccharides (LPS); or IPS analog (MPLA) | Gram-negative bacteria | AGP |
| | Heat shock protein | Bacteria and host cell | MPLA |
| | Fibrinogen | Host cell | RC-529 |
| | Heparin sulfate fragments | Host cell | MDF2B |
| | Hyaluronic acid fragments | Host cell | CFA |
| | Nickel | | |
| | Various opoid drugs | | |
| | Protein | Mycobacteriu m tuberculosis | RpfE, RpfB, Rv2299c, HBHA |
| TLR5 | Flagellin | Bacteria | Flagellin |
| TLR6 | Multiple diacyl lipopeptides | Mycoplasma | FSL1-** |
| | | | Pam2Cys** |
| | | | MALP2-* * |
| TLR7 | Virus ssRNA (influenza, VSV, HIV, HCV) | RNA virus | Guanosine analogs; imidazoquino lines (e.g. Imiquimod, Aldara ® R848, Resiquimod)®), loxoribine |
| TLR8 | Small synthetic compounds; single-stranded RNA | RNA, human and virus | Imidazoquinolines ; loxoribine; ssPolyU, 3M-012 |
| TLR9 | Unmethylated CpG Oligodeoxynucleotide DNA; DNA; dsDNA virus (HSV, MCMV); Hemozoin (Plasmodium) | Bacteria, DNA virus | CpG-oligonucleotides, various sequences as synthesized (e.g. CpG-ODN 2006, 1826, 2395) |
| TLR10 | | | |
| TLR11 | Profilin | Toxoplasma gondii | |
| TLR12 | Profilin | Toxoplasma gondii | |
| TLR13 | Bacterial ribosomal RNA sequence | Virus, bacteria | |
| | "CGGAAAGACC" | | |
| * Ligands recognized by TLR1 and TLR2 | | | |
| ** Ligands recognized by TLR2 and TLR6 | | | |

In accordance with the present disclosure, the toll-like receptor agonist may be derived from microorganisms, viruses, plants or animals, or may be synthesized. There is no particular restriction on a source thereof.

Further, the differentiation of the immature dendritic cells in accordance with the present disclosure may be performed using the differentiation-inducing factor. In this connection, it is preferable to use the FMS-like tyrosine kinase 3 ligand (Flt3L) as the differentiation-inducing factor. The differentiation-inducing factor is not particularly limited as long as the differentiation-inducing factor can induce the differentiation of the immature dendritic cells into plasmacytoid dendritic cells and conventional dendritic cells (conventional plasmacytoid cells, cDCs).

Further, in accordance with the present disclosure, the differentiation of the immature dendritic cells may be accomplished by incubating the immature dendritic cells in a medium containing the differentiation-inducing factors, by adding the differentiation-inducing factor to the culture medium for the immature dendritic cells.

Further, in accordance with the present disclosure, optionally, the differentiation-inducing factor may be added one or more times during the differentiation of the immature dendritic cells.

As used herein, the term, "FMS-like tyrosine kinase 3 (Flt3L)" refers to an endogenous small molecule that acts as a cytokine and growth factor by activating hematopoietic progenitors.

Further, in accordance with the present disclosure, the duration of differentiation of the immature dendritic cells is not particularly limited. The duration of differentiation of the immature dendritic cells may vary depending on the type of medium to be used and the environment. For example, the duration of differentiation of the immature dendritic cells may be in range from 5 days to 15 days, preferably from 7 days to 10 days, more preferably from 8 days to 9 days.

In accordance with the present disclosure, immune tolerance of the plasmacytoid dendritic cells may be activated by further treating the toll-like receptor agonist to the tolerogenic plasmacytoid dendritic cells differentiated in an induced manner from the immature dendritic cells by treating the immature dendritic cells using the toll-like receptor agonist.

In accordance with the present disclosure, the types of toll-like receptor agonist used to activate the tolerogenic plasmacytoid dendritic cells differentiated as described above are not particularly limited but may include at least one selected from a group consisting of a TLR1 agonist, a TLR2 agonist, a TLR3 agonist, a TLR4 agonist, a TLR5 agonist, a TLR6 agonist, a TLR7 agonist, a TLR8 agonist, a TLR9 agonist, a TLR11 agonist, a TLR12 agonist, and a TLR13 agonist. Preferably, the TLR9 agonist may be used.

In accordance with the present disclosure, the differentiation of the immature dendritic cells into the tolerogenic plasmacytoid dendritic cells may be induced through the above process. Therefore, it is possible to stably supply a large amount of the tolerogenic plasmacytoid dendritic cells via the simple and easy process using the immature dendritic cells.

According to another implementation of the present disclosure, the present disclosure relates to the tolerogenic plasmacytoid dendritic cells produced by the method.

The tolerogenic plasmacytoid dendritic cells obtained from the present disclosure can induce differentiation of regulatory T cells and inhibit the activity of effector T cells, thereby effectively suppressing the immune response.

Further, the present disclosure relates to cell therapeutic agents including the tolerogenic plasmacytoid dendritic cells.

As used herein, the term, "cell therapeutic agent" refers to a medical product for treatment, diagnosis, and prevention via a series of actions including proliferating and selecting alive autologous, allogenic, xenogenic cells *in vitro*, or changing the biological characteristics of the cell with other methods in order to restore cell and tissue function. The United States has classified the cell therapeutic agent as pharmaceuticals since 1993, and Korea since 2002. These cell therapeutic agents may be categorized into two categories. A first category includes immune cell therapeutic agents for the control of immune responses such as inhibition of *in vivo* immune response or exacerbation of the immune response. The second category includes a stem cell therapeutic agent for tissue regeneration or organ function recovery. The cell therapeutic agent provided in accordance with the present disclosure may be the immune cell therapeutic agent.

In accordance with the present disclosure, the cell therapeutic agent may be used to induce differentiation of regulatory T cells or stimulate activity thereof and may be used to prevent or treat autoimmune diseases, hypersensitivity immune diseases and various allergic diseases, in particular, by effectively suppressing the immune response.

In accordance with the present disclosure, the administration route of the cell therapeutic agent may be any conventional route as long as it can reach the target tissue. The administration route may include parenteral administration, for example, intraperitoneal administration, intravenous administration, intramuscular administration, subcutaneous administration, intravenous administration. However, the present disclosure is not limited thereto. The compositions may be formulated in a suitable form together with a pharmaceutical carrier commonly used in cell therapy. The term "pharmaceutically acceptable carrier" refers to compositions which are physiologically acceptable and which, when administered to humans, do not normally cause allergic reactions such as gastrointestinal disorders, dizziness, or the like. The pharmaceutically acceptable carriers include, for example, carriers for parenteral administration such as water, suitable oils, saline, aqueous glucose and glycols, etc., and may further include stabilizers and preservatives. The suitable stabilizers include antioxidants such as sodium hydrogen sulfite, sodium sulfite or ascorbic acid. Suitable preservatives include benzalkonium chloride, methyl- or propyl-paraben and chlorobutanol.

Further, in accordance with the present disclosure, the cell therapeutic agent may be administered by any device capable of migrating to a target cell.

The cellular therapeutic agent in accordance with the present disclosure may include a therapeutically effective amount of a cell therapeutic agent for the treatment of the disease. The therapeutically effective amount refers to the amount of active ingredient or pharmaceutical composition that induces a biological or medical response in a tissue system, animal or human, as contemplated by a researcher, veterinarian, physician or other clinicians. This includes the amount that induces the relief of the symptoms of the disease or disorder being treated. It is apparent to those skilled in the art that the content (number) of plasmacytoid dendritic cells included in the cell therapeutic agent in accordance with the present disclosure will vary depending on the desired effect. Therefore, the optimum cell therapeutic agent content may be easily determined by those skilled in the art and may be controlled based on various factors such as the type of the disease, the severity of the disease, the content of the other ingredients contained in the composition, the type of formulation, and the age, weight, general health status, sex and diet of a patient, time of administration, route of administration and secretion rate of the composition, duration of treatment, a type of a drug as administrated concurrently, etc. For example, the plasmacytoid dendritic cells of 2 × 10⁴ cells/ml to 8 × 10⁷ cells/ml may be included in the cell therapeutic agent. However, the present disclosure is not limited thereto.

In the method of treatment in accordance with the present disclosure, the composition including the cellular therapeutic agent in accordance with the present disclosure as an active ingredient may be administered via any of a variety of routes including rectal, intravenous, intraarterial, intraperitoneal, intramuscular, intrasternal, percutaneous, local, intraocular subcutaneous or intracutaneous routes.

The cell therapeutic agent in accordance with the present disclosure that includes the tolerogenic plasmacytoid dendritic cells as an active ingredient may be used as an external preparation for skin to prevent or ameliorate autoimmune diseases, hypersensitivity immune diseases or allergic diseases. In this case, the formulation thereof depending on the body part is not particularly limited. Specifically, the external preparation may be, for example, a cosmetic composition having a formulation of a softening agonist, a nutritional lotion, a massage cream, a nutritional cream, a pack, a gel or a skin adhesive type cosmetic. Further, the external preparation may be a transdermal dosage form such as lotion, ointment, gel, cream, patch or spray. Further, in the composition for external application according to each formulation, components other than the tolerogenic plasmacytoid dendritic cells in accordance with the present disclosure may be selected and mixed therewith without difficulty by those skilled in the art depending on the formulation or use purpose of other external preparations for skin. In this case, a synergistic effect may occur when the components other than the tolerogenic plasmacytoid dendritic cells are mixed with the tolerogenic plasmacytoid dendritic cells.

Hereinafter, the present disclosure will be described in more detail with reference to Examples. It should be understood by those skilled in the art that these Examples are only for describing the present disclosure more specifically and that the scope of the present disclosure is not limited to these Examples and is in accordance with the gist of the present disclosure.

### Examples

### [Example 1] Regulatory effect of differentiation into plasmacytoid dendritic cells by toll-like receptor agonist

In the treatment of the immature dendritic cells using the toll-like receptor agonists during differentiation of the immature dendritic cells, in order to check whether the immature dendritic cells are differentiated in an induced manner into the tolerogenic plasmacytoid dendritic cells, the following experiment was carried out according to the design diagram shown in FIG. 1.

Specifically, thigh bone marrow samples were collected from C57BL/6 mice using bone marrow collecting syringes. The collected bone marrow was washed with phosphate buffered saline (PBS), and red blood cells were removed therefrom using ammonium chloride. Separated cells (3 × 10⁶ cells/well) were inoculated in a 6-well plate. Then, 1 ml of RPMI 1640 containing 10% FBS (fetal bovine serum), 2 mM L-glutamine, 100 U/ml penicillin/streptomycin, 50 µM mercaptoethanol, 0.1 mM non-essential amino acid, 1 mM sodium pyruvate and 250 ng/ml FLT3L was added thereto to initiate culture and differentiation of the cells. On the third day after the start of differentiation, Pam3 was applied thereto at a concentration of 100 to 500 ng/ml. On 5 days from the start of differentiation, 1 ml of the RPMI 1640 was further supplemented thereto. The cells were cultured for 8 days. From the cells obtained after the incubation, plasmacytoid dendritic cells were separated at a purity equal to or greater than 85% by a plasmacytoid dendritic cell separation kit (Miltenyi Biotec, Auburn, CA) and a magnetic cell sorting system (Vario MACS: Miltenyi Biotec, Auburn, CA) (FIG. 2). Because the treatment using the toll-like receptor agonist may affect the differentiation into plasmacytoid dendritic cells, surface expression molecules specifically induced from the plasmacytoid dendritic cells were checked on the eighth day and the results are shown in FIG. 3A. Further, in the case (TLRs-pDC) when the immature dendritic cells were treated with the toll-like receptor agonist, the ratio of the number of plasmacytoid dendritic cells obtained after the eighth day to the number of the initial immature dendritic cells was measured. The results are shown in FIG. 3B. In this connection, in order to check the differentiation regulatory effect of the toll-like receptor agonist, the case without the toll-like receptor agonist-based treatment is shown as Comparative Example (pDC).

As shown in FIG. 2, we were able to confirm that the differentiation into the plasmacytoid dendritic cells was induced by applying the toll-like receptor agonist to the immature dendritic cells. We could confirm that there is no difference between differentiation efficiency in the non-treatment using the toll-like receptor agonist and differentiation efficiency in the treatment using the toll-like receptor agonist.

### [Example 2] Stimulus to isolated plasmacytoid dendritic cells and confirmation of secretion pattern of cytokine therefrom

In order to check the cytokine secretion pattern from the plasmacytoid dendritic cells as isolated from Example 1, the isolated cells (5 × 10⁵ cells/ml) were inoculated into a 48 well plate and treated and stimulated with ODN1826 (1 µg/ml). At 24 hours after the stimulation, supernatant was separated, and the cytokine secretion pattern was checked by ELISA (enzyme linked immunosorbent assay) and the results are shown in FIG. 4. In this connection, in order to check the effect from the treatment using the toll-like receptor agonist, in Example 1, the case in which the toll-like receptor agonist was not applied thereto during the differentiation of the immature dendritic cells is shown as the Comparative Example (pDC).

As shown in FIG. 4, in general, a type I interferon (IFN-α and IFN-β) and the typical inflammatory cytokines TNF-α and IL-12p70 were strongly induced from the plasmacytoid dendritic cells (pDC). However, when the plasmacytoid dendritic cells (TLRs-pDC) differentiated in the induced manner via the treatment with the toll-like receptor agonist (0.5 µg/ml) were stimulated with ODN1826, the expression level of the type I interferon (IFN-α and IFN-β) and the typical inflammatory cytokines TNF-α and IL-12p70 was greatly reduced. To the contrary, when the plasmacytoid dendritic cells (TLRs-pDC) differentiated in the induced manner via the treatment with the toll-like receptor agonist (0.5 µg/ml) were stimulated with ODN1826, the expression level of IL-10 as an immunosuppressive cytokine was significantly increased.

In this way, it has been shown that the plasmacytoid dendritic cells differentiated in the induced manner from the immature dendritic cells via applying the toll-like receptor agonist to the immature dendritic cells according to the present disclosure have immune tolerance unlike the conventional plasmacytoid dendritic cells.

### [Example 3] Confirmation of IL-10 secretion pattern from plasmacytoid dendritic cells induced via treatment using various toll-like receptor agonists

In order to check the immune tolerance-inducing effect of various toll-like receptor agonists, differentiation into the plasmacytoid dendritic cells was induced by the same method as in Example 1. Ligands shown in Table 2 were used as the toll-like receptor agonist. From the cells obtained after 8 days of culture, the plasmacytoid dendritic cells were separated at a purity equal to or greater than 85% by a plasmacytoid dendritic cell separation kit (Miltenyi Biotec, Auburn, CA) and a magnetic cell sorting system (Vario MACS: Miltenyi Biotec, Auburn, CA). The separated cells (5 × 10⁵ cells/ml) were inoculated into a 48 well plate. ODN1826 (1 µg/ml) was applied thereto and the cells were cultured for 24 hours. Then, supernatant was separated. The secretion pattern of the immunosuppressive cytokine IL-10 was then checked by ELISA (enzyme linked immunosorbent assay). The results are shown in FIG. 5. In this connection, in order to check the effect from the treatment using the toll-like receptor agonist, the case in which the toll-like receptor agonist was not applied thereto during the differentiation of the immature dendritic cells is shown as Comparative Example (non).

**[Table 2]**

| Non | | TLR2 agonist | TLR3 agonist | TLR4 agonist | TLR7 agonist | TLR9 agonist |
|---|---|---|---|---|---|---|
| Non-treatment | | Pam3 | Poly I : C | LPS | Imiquimod | CpG-ODN |

As shown in FIG. 5, the expression level of IL-10 from only the plasmacytoid dendritic cells differentiated in the induced manner via treatments with TLR2, TLR4, TLR7 and TLR8 agonists which affects MYD88 signal among various toll-like receptor agonists was significantly increased when the plasmacytoid dendritic cells were stimulated with ODN1826.

In this case, only the treatment using the toll-like receptors which affects the MYD88 signal during differentiation of the immature dendritic cells may induce the tolerogenic plasmacytoid dendritic cells.

### [Example 4] Confirmation of cytokine secretion pattern from plasmacytoid dendritic cells according to treatment time using toll-like receptor agonist

Differentiation into the plasmacytoid dendritic cells was induced by the same method as in Example 1 in order to check the immune tolerance-inducing effect of the toll-like receptor agonist according to the treatment time point. Further, the toll-like receptor agonists were applied at the start time of the differentiation (0 day treatment) and were applied on 3 days after the start of differentiation (3 day treatment). After the total 8 days culturing, the plasmacytoid dendritic cells were isolated therefrom at a purity equal to or greater than 85% using a plasmacytoid dendritic cell separation kit (Miltenyi Biotec, Auburn, CA) and a magnetic cell sorting system (Vario MACS: Miltenyi Biotec, Auburn, CA). The separated cells (5 × 10⁵ cells/ml) were inoculated into a 48 well plate. ODN1826 (1 µg/ml) was applied thereto and the cells were cultured for 24 hours. Then, supernatant was separated. The secretion pattern of the cytokine was then checked by ELISA (enzyme linked immunosorbent assay). The results are shown in FIG. 6. In this connection, in order to check the effect from the treatment using the toll-like receptor agonist, the case in which the toll-like receptor agonist was not applied thereto during the differentiation of the immature dendritic cells is shown as Comparative Example (no-treatment, non).

As shown in FIG. 6, when the toll-like receptor agonist was applied at the start of differentiation (0 day treatment) or 3 days (3 days treatment) thereafter, the expression level of the type I interferon (IFN-α and IFN-β) and TNF-α and IL-12p70 as the most prominent inflammatory cytokines were inhibited as compared with the no-treatment, but the expression level of IL-10 as an anti-inflammatory cytokine was significantly increased as compared with the no-treatment.

Thus, even when the toll-like receptor agonist was applied simultaneously together with the differentiation-inducing factor FLT3L, that is, when the toll-like receptor agonist was applied at the start time of differentiation of the immature dendritic cells, the differentiation into the tolerogenic plasmacytoid dendritic cells was induced as in the case of the treatment using the toll-like receptor agonist during differentiation of the immature dendritic cells.

### [Example 5] Confirmation of expression of co-stimulation factor and MHC molecule from plasmacytoid dendritic cells induced via treatment with toll-like receptor agonist

Dendritic cells including the plasmacytoid dendritic cells may present an external antigen via MHC molecules to activate T cells when detecting the external antigen and may express co-stimulation factors such as CD80 and CD86 to stimulate interactions.

Thus, the plasmacytoid dendritic cells isolated from Example 1 were stimulated with ODN1826, and then cultured for 24 hours. Then, the expression levels of the cell surface molecules therefrom were checked. To investigate the effect of the plasmacytoid dendritic cells on the surface factor expression, anti-CD11c (PE-Cy7, BD Biosciences), and anti-PDCA-1 (PerCP-eFluor 710, ebioscience) antibodies as markers specific to the plasmacytoid dendritic cells, and anti-CD80 (v450, BD Biosciences), anti-CD86 (APC, ebioscience), anti-MHC-I (PE, ebioscience), and anti-MHC-II (APC-eFluor 780, ebioscience) antibodies as markers specific to the cell surface molecules were applied thereto at 4°C for 30 minutes. Then, the treated plasmacytoid dendritic cells were analyzed using a flow cytometry device LSRFortessa x-20 (BD Biosciences). The results are shown in FIG. 7. In this connection, in order to check the effect from the treatment using the toll-like receptor agonist, the case in which the toll-like receptor agonist was not applied thereto during the differentiation of the immature dendritic cells is shown as the Comparative Example (pDC).

As shown in FIG. 7, co-stimulation factor CD86 and MHC class II molecule presenting the exogenous antigen were expressed at high levels from the conventional plasmacytoid dendritic cells (pDC). However, co-stimulation factor CD86 and MHC class II molecule presenting the exogenous antigen were not expressed or were expressed at a lower level from the plasmacytoid dendritic cells (TLRs-pDC) induced via treatment with toll-like receptor agonist. To the contrary, one of other co-stimulatory factors, CD80 and MHC class I molecules presenting the endogenous antigen or cross-antigen were expressed at high levels from the plasmacytoid dendritic cells (TLRs-pDC) induced via treatment with toll-like receptor agonist, compared to the conventional plasmacytoid dendritic cells (pDC) without treatment with toll-like receptor agonist.

### [Example 6] Confirmation of expression of immune tolerance-inducing molecule from plasmacytoid dendritic cells induced via treatment with toll-like receptor agonist

The conventional dendritic cells activate the acquired immune response by inducing T cell response via antigen presenting. However, some tolerogenic dendritic cells have been reported to suppress T cell responses. The inhibition of the immune responses has recently been reported to be achieved by the induction of various immune tolerance-inducing molecules, for example, PD-L1 and IDO. Further, CCR9 + pDCs have been reported to induce various immune tolerance phenomena via strong induction of regulatory T cells.

Therefore, in order to check the expression of immune tolerance-inducing molecules from the plasmacytoid dendritic cells isolated from Example 1, anti-CDllc (PE-Cy7, BD Biosciences), and anti-PDCA-1 (PerCP-eFluor 710, ebioscience) antibodies as markers specific to the plasmacytoid dendritic cells, and anti-PD-Ll (PE, ebioscience), and anti-CCR9 (FITC, ebioscience) as immune tolerance-inducing cell surface molecules were applied thereto at 4°C for 30 minutes. Further, to determine the expression of IDO induced in the cell, fixation/permeabilization substance (BD Bioscience) was applied thereto at 4°C for 30 minutes, and anti-IDO (eFluor 660, ebioscience) was stained. The expression levels thereof were analyzed using a flow cytometer LSRFortessa x-20 and the results are shown in FIG. 8. In this connection, in order to check the effect from the treatment using the toll-like receptor agonist, the case in which the toll-like receptor agonist was not applied thereto during the differentiation of the immature dendritic cells in Example 1 is shown as the Comparative Example (pDC).

As shown in FIG. 8, when the plasmacytoid dendritic cells induced via the treatment with the toll-like receptor agonist were stimulated with ODN1826, the expression levels of CCR9 and IDO molecules therefrom were significantly increased compared with the conventional plasmacytoid dendritic cells (pDC) without treatment. When the plasmacytoid dendritic cells induced via the treatment with the toll-like receptor agonist were not stimulated with ODN1826, the expression level of CCR9 molecules therefrom was higher compared with the conventional plasmacytoid dendritic cells (pDC) without treatment.

Thus, in the treatment using the toll-like receptor agonists during differentiation of the immature dendritic cells, the differentiation into the plasmacytoid dendritic cells having immune tolerance may be induced.

### [Example 7] Confirmation of regulatory T cell-inducing ability by plasmacytoid dendritic cells induced via treatment using toll-like receptor agonist

Tolerogenic plasmacytoid dendritic cells (Tolerogenic pDCs) have been reported to induce differentiation into regulatory T cells (Treg) and inhibit the activity or proliferation of effector T cells.

Therefore, we examined whether the regulatory T cells (Foxp3 + CD4 + T cells) are proliferated by the plasmacytoid dendritic cells (TLRs-pDC) differentiated in the induced manner via treatment using the toll-like receptor agonist. Specifically, T cells isolated from allogeneic mice and then stained with CellTrace (Invitrogen) were mixed and cultured with the plasmacytoid dendritic cells (TLRs-pDC) isolated from Example 1 at a mixing ratio of 5 : 1 for 5 culturing days. The plasmacytoid dendritic cells stimulated with ODN1826 (ODN +) and the plasmacytoid dendritic cells not stimulated with ODN1826 (ODN -) were used as the plasmacytoid dendritic cells. After 5 days of culture, anti-CD4 (Percp-cy5.5, ebioscience) was applied thereto at 4°C for 30 minutes and then Foxp3/transcription factor staining buffer (eBioscience) was applied thereto at 37°C for 30 minutes. After the treatment with anti-Foxp3 (PE, ebioscience), the results were checked by the flow cytometer LSRFortessa x-20 for proliferation into the regulatory T cell. FIG. 9A shows the results. The proliferation rate of regulatory T cells was calculated and the results are shown in FIG. 9B._In this connection, in order to check the effect from the treatment using the toll-like receptor agonist, the plasmacytoid dendritic cells stimulated with ODN1826 are indicated as ODN + while conventional plasmacytoid dendritic cells not stimulated with ODN1826 are indicated as Comparative Example (pDC).

As shown in FIG. 9, the conventional plasmacytoid dendritic cells (pDC) did not induce the regulatory T cell proliferation. However, when the conventional plasmacytoid dendritic cells were subjected to the stimulation with ODN1826 (ODN +), this resulted in the inhibition of regulatory T cell proliferation. To the contrary, when the plasmacytoid dendritic cells (TLRs-pDC) differentiated in the induced manner via the toll-like receptor agonist based treatment were subjected to the stimulation with ODN1826 (ODN +), this induced the proliferation of regulatory T cell more significantly than when the plasmacytoid dendritic cells (TLRs-pDC) differentiated in the induced manner via the toll-like receptor agonist based treatment were not subjected to the stimulation with ODN1826 (ODN -).

Thus, when the plasmacytoid dendritic cells (TLRs-pDC) differentiated in the induced manner according to the present disclosure were subjected to further toll-like receptor agonist based treatment, the tolerogenic activity was induced and differentiation into the regulatory T cells was further induced.

### [Example 8] Confirmation of activity of Rv1411c protein as toll-like receptor agonist

Previous reports have shown that proteins derived from *M. tuberculosis* can bind to various toll-like receptors. Thus, the binding of Rv1411c protein to dendritic cells differentiated from the bone marrow of TLR2 knockout mice and wild-type mice was checked to check the activity of Rv1411c protein as a toll-like receptor agonist.

Specifically, mouse bone marrow was separated from the mouse and red blood cells were removed therefrom. For differentiation into the dendritic cells, the bone marrow was cultured for 8 days in RPMI 1640 medium containing 10% FBS, 1% antibiotic and 100 ng/ml GM-CSF (granulocyte-macrophage-colony stimulating factor). After 8 days of culture, 5 µg/ml of Rv1411c protein was applied to each of the wild type dendritic cells (WT) and dendritic cells isolated from TLR2 knockout mice (TLR2 -/-) and then intermittently mixed with each other for 2 hours, to facilitate the binding of the protein to the dendritic cells. Then, after staining the mixture using antibodies having antigen-antibody specificity to His molecules labeled to the Rv1411c protein, the degree of binding therebetween was measured using a flow cytometer. The results are shown in FIG. 10.

As shown in FIG. 10, the wild-type dendritic cells (WT) were found to have increased binding to the Rv1411c protein compared to TLR2 knockout-derived dendritic cells (TLR2 -/-). When the TLR2 knockout-derived dendritic cells (TLR2 -/-) were treated using the Rv1411c protein, the binding strength therebetween was confirmed to be the same as that in the untreated experimental group.

Thus, it was confirmed that the Rv1411c protein binds to the TLR2 receptor and has activity as the TL2 agonist.

### [Example 9] Inducing ability of differentiation into tolerogenic plasmacytoid dendritic cells by Rv1411c protein

Differentiation into the tolerogenic plasmacytoid dendritic cells was induced by the same procedure as in Example 1. The toll-like receptor agonist employed the Rv1411c protein (0.1 µg/ml, 0.5 µg /ml), whose activity as the toll-like receptor agonist was confirmed in Example 8. After 8 days of culture, plasmacytoid dendritic cells were separated at a purity of 85% or greater using a plasmacytoid dendritic cell separation kit (Miltenyi Biotec, Auburn, CA) and a magnetic cell sorting system (Vario MACS: Miltenyi Biotec, Auburn, CA). The separated cells (5 × 10⁵ cells/ml) were inoculated into a 48 well plate. After treatment of the cells with ODN1826 (1 µg/ml), the cells were cultured for 24 hours. After culturing, the supernatant was separated and then the cytokine secretion pattern was checked via Enzyme-Linked Immunosorbent Assay (ELISA). The result is shown in FIG. 11. In this connection, in order to check the effect from the treatment using the Rv1411c protein, the plasmacytoid dendritic cells treated with Rv1411c protein is indicated as Rv1411c-pDC, and the conventional plasmacytoid dendritic cells stimulated with ODN1826 (ODN +) and not stimulated with ODN1826 (ODN -) are indicated as Comparative Example (pDC).

As shown in FIG. 11, when the plasmacytoid dendritic cells (Rv1411c (0.1 µg /ml)-pDC, Rv1411c (0.5 µg /ml)-pDC) treated with the Rv1411c protein were stimulated with ODN1826, the expression of the type I interferon (IFN-α and IFN-β) and TNF-α and IL-12p70, as typical inflammatory cytokines as strongly induced from the plasmacytoid dendritic cells was drastically reduced in a concentration-dependent manner, while the expression level of IL-10, as an immunosuppressive cytokine has a marked increase in a concentration-dependent manner.

These results suggest that plasmacytoid dendritic cells treated with Rv1411c protein have the immune tolerance which is not the case for conventional plasmacytoid dendritic cells. The level of the immune tolerance was increased in proportion to the concentration of the Rv1411c protein.

### [Example 10] Confirmation of expression of co-stimulation factor, MHC molecule and immune tolerance inducing molecule from plasmacytoid dendritic cells differentiated in the induced manner by treatment with Rv1411c protein

In Example 9, the expression patterns of the surface molecules and enzymes from the plasmacytoid dendritic cells differentiated in the induced manner via the treatment using the Rv1411c protein were checked.

First, to investigate the effect of the plasmacytoid dendritic cells on the surface factor expression, anti-CD11c (PE-Cy7, BD Biosciences), and anti-PDCA-1 (PerCP-eFluor 710, ebioscience) antibodies as markers specific to the plasmacytoid dendritic cells, and anti-CD80 (v450, BD Biosciences), anti-CD86 (APC, ebioscience), anti-MHC-I (PE, ebioscience), anti-MHC-II (APC-eFluor 780, ebioscience), anti-PD-L1(PE, ebioscience) and anti-CCR9 (FITC, ebioscience) antibodies as markers specific to the cell surface factors were applied thereto at 4°C for 30 minutes. Further, to determine the expression of IDO induced in the cell, fixation/permeabilization substance (BD Bioscience) were applied thereto at 4°C for 30 minutes, and anti-IDO (eFluor 660, ebioscience) was stained after treatment. The expression levels thereof were analyzed using a flow cytometer LSRFortessa x-20 and the results are shown in FIG. 12. In this connection, in order to check the effect from the treatment using the Rv1411c protein, the plasmacytoid dendritic cells treated with Rv1411c protein is indicated as Rv1411c-pDC, and the conventional plasmacytoid dendritic cells stimulated with ODN1826 (ODN +) and not stimulated with ODN1826 (ODN -) are indicated as Comparative Example (pDC) as non-treated with the Rv1411c protein.

As shown in FIG. 12, the co-stimulatory factor CD86 and MHC class II molecule presenting exogenous antigen are expressed at high levels from the conventional plasmacytoid dendritic cells (pDC) as non-treated with the Rv1411c protein during the ODN1826 stimulus thereto. However, the co-stimulatory factor CD86 and MHC class II molecule presenting exogenous antigen are expressed at very lower levels or are not expressed from the plasmacytoid dendritic cells (Rv141Ic-pDC) as differentiated in the induced manner via the treatment with the Rv1411c protein during the ODN1826 stimulus thereto.

To the contrary, CD80 as one of the other co-stimulatory factors, and an MHC class I molecule presenting the endogenous or cross-antigen was expressed at higher levels from the plasmacytoid dendritic cells (Rv1411c-pDC) as differentiated in the induced manner via the treatment with the Rv1411c protein than from the conventional plasmacytoid dendritic cells (pDC).

Further, the expression levels of PD-L1, CCR9, and IDO molecules from the plasmacytoid dendritic cells (Rv1411c-pDC) as differentiated in the induced manner via the treatment with the Rv1411c protein were significantly increased during the ODN1826 stimulus thereto (ODN+) compared to the ODN1826 non-stimulus (ODN-). Further, the expression level of the CCR9 and PD-L1 from the plasmacytoid dendritic cells (Rv1411c-pDC) as differentiated in the induced manner via the treatment with the Rv1411c protein during the ODN1826 non-stimulus thereto (ODN-) are higher than the expression levels thereof from the conventional plasmacytoid dendritic cells (pDC).

### [Example 11] Inhibition of T cell activation by plasmacytoid dendritic cells differentiated in the induced manner by treatment with Rv1411c protein

The most important feature of the tolerogenic plasmacytoid dendritic cells in vivo is to inhibit the activity of T lymphocytes. The following experiment was conducted to check the effect of the plasmacytoid dendritic cells differentiated in the induced manner via the treatment using the Rv1411c protein in Example 9 on the proliferation and activity of T cells.

Specifically, T cells (1.5 × 10⁵ cells/well) as isolated from allogeneic mice and stained with a CellTrace (Invitrogen) were stimulated with 1 × PMA/Ionomycin (ebioscience). This treatment of the PMA/Ionomycin increases the proliferation rate of T lymphocytes, thereby promoting the secretion of interferon gamma (IFN-gamma). When these reactions were carried out, T cells were mixed at a mixing ratio of 1 : 5 with the plasmacytoid dendritic cells (Rv1411c-pDC) differentiated in the induced manner via the treatment using the Rv1411c protein or the conventional plasmacytoid dendritic cells (pDC) and then the mixture was cultured for 3 days. After 3 days, anti-CD4 (Percp-cy5.5, ebioscience) and anti-CD8 (Percp-cy5.5, ebioscience) were applied thereto at 4°C for 30 minutes. The treated mixture was stained. The proliferation of T cells was analyzed by a flow cytometer LSRFortessa x-20 and the results are shown in FIG. 13. Further, the supernatant obtained after 3 days of culture was separated, and then the secretion pattern of IFN-gamma was checked via an Enzyme-Linked Immunosorbent Assay (ELISA). In this connection, in order to check the effect from the treatment using the Rv1411c protein, the plasmacytoid dendritic cells treated with Rv1411c protein is indicated as Rv1411c-pDC, and the conventional plasmacytoid dendritic cells stimulated with ODN1826 (ODN +) and not stimulated with ODN1826 (ODN -) are indicated as Comparative Example (pDC) as non-treated with the Rv1411c protein.

As shown in FIG. 13, both the plasmacytoid dendritic cells (Rv1411c-pDC) differentiated in the induced manner via the treating using the Rv1411c protein and the conventional plasmacytoid dendritic cells (pDC) induced T cell proliferation. However, the plasmacytoid dendritic cells (Rv1411c-pDC) differentiated in the induced manner via the treating using the Rv1411c protein has the lower induction ability of the proliferation of CD4 + T cells compared to that of the conventional plasmacytoid dendritic cells (pDC).

In addition, as shown in FIG. 14, the plasmacytoid dendritic cells (Rv1411c-pDC) differentiated in the induced manner via the treating using the Rv1411c protein has a lower level of secretion of IFN-gamma compared to that of the non-treated conventional plasmacytoid dendritic cells (pDC).

### [Example 12] Control of regulatory T cell differentiation by plasmacytoid dendritic cells differentiated in induced manner via treating using Rv1411c protein

T cells as isolated from allogeneic mice and stained with a CellTrace (Invitrogen) were mixed at a mixing ratio of 10 : 1, 5 : 1 or 1 : 1 with the plasmacytoid dendritic cells (Rv1411c-pDC) differentiated in the induce Ò manner via the treatment using the Rv1411c protein or the conventional plasmacytoid dendritic cells (pDC) and then the mixture was cultured for 5 days. In this connection, the plasmacytoid dendritic cells were stimulated with ODN1826 (ODN +) and unstimulated with ODN1826 (ODN -). On the fifth day after the incubation, anti-CD4 (Percp-cy5.5, ebioscience) and anti-CD4 (Percp-cy5.5, ebioscience) were applied thereto at 4° C for 30 minutes. Then, anti-Foxp3/transcription factor staining buffer (ebioscience) was applied thereto at 37°C for 30 minutes. Then, the mixture was stained with anti-Foxp3 (PE, ebioscience). The proliferation into the regulatory T cells was checked using flow cytometer LSRFortessa x-20. The results are shown in FIG. 15(a). Further, the proliferation rate of regulatory T cell was calculated and the results are shown in FIG. 15(b).

As shown in FIG. 15, the conventional plasmacytoid dendritic cells (pDC) did not induce regulatory T cell proliferation. When the stimulation with ODN1826 was applied to pDC, this leads to the inhibition of regulatory T cell proliferation. To the contrary, when the plasmacytoid dendritic cells (Rv1411c-pDC) differentiated in the induced manner via treatment with Rv1411c protein was subjected to the stimulation with ODN1826 (ODN +), this induces proliferation of regulatory T cell more significantly than when the plasmacytoid dendritic cells (Rv1411c-pDC) was unstimulated (ODN -). As the ratio of the plasmacytoid dendritic cells (Rv1411c-pDC) to T cells increased, we could check that the proliferation rate of regulatory T cells further increased.

Thus, the toll-like receptor agonist was further applied to the tolerogenic plasmacytoid dendritic cell differentiated in the induced manner in accordance with the present disclosure, the tolerogenic capacity may be activated to allow the regulatory T cell proliferation to be further effectively induced.

### [Example 13] Inhibition of effector T cell activity by plasmacytoid dendritic cells differentiated in the induced manner by treatment with Rv1411c protein

Regulatory T cells have been reported to induce the tolerogenic dendritic cells and inhibit the proliferation of other T cells. Therefore, we examined the activity of the plasmacytoid dendritic cells differentiated in the induced manner by the Rv1411c protein-based treatment according to Example 12 as a regulatory T cell.

First, the spleen of the mouse is separated and the red blood cells are removed therefrom. CD4 +, CD25-effector T cells were isolated using a magnetic cell sorting system (MACS) and stained with a violet proliferation dye to determine the degree of proliferation. Then, the differentiated T cells were incubated together with CD4 +, CD25-effector T cells for 2 days in wells coated with anti-CD3e and anti-CD28 antibodies. As a result, the change in T cell proliferation ability was measured based on the ratio of primed T cells to CD25-effector T cells. The results are shown in FIG. 16.

As shown in FIG. 16, the T cells induced only by the ODN1826-stimulated cells (Rv1411c-pDC (ODN)) among the plasmacytoid dendritic cells differentiated in the induced manner by the Rv1411c protein-based treatment reduced the level of differentiation of effector T cells.

Thus, when the toll-like receptor agonist was further applied to the tolerogenic plasmacytoid cell differentiated in accordance with the present disclosure, the tolerogenic ability may be activated to allow the effector T cell activation to be effectively inhibited.

### [Example 14] Acquisition yield of plasmacytoid dendritic cells differentiated in induced manner by treatment using toll-like receptor agonist

Thigh bone marrow was collected from C57BL/6 mice using a bone marrow collecting syringe. The collected bone marrow was washed with phosphate buffered saline (PBS), and red blood cells were removed therefrom using ammonium chloride. Separated cells (5 × 10⁵ cells/well) were inoculated in a 6-well plate. Then, 1 ml of RPMI 1640 containing 10% FBS (Fetal bovine serum), 2 mM L-glutamine, 100 U/ml penicillin/streptomycin, 50 µM mercaptoethanol, 0.1 mM non-essential amino acid, 1 mM sodium pyruvate and 250 ng/ml FLT3L was added thereto to initiate culture and differentiation of the cells. On the third day after the start of differentiation, Pam3 and Rv1411c protein as the toll-like receptor agonist were applied thereto at a concentration of 100 to 500 ng/ml. On 5 days from the start of differentiation, 1 ml of the RPMI 1640 was further supplemented thereto. The cells were cultured for 8 days. From the cells obtained after the incubation, plasmacytoid dendritic cells were separated at a purity equal to or greater than 85% by a plasmacytoid dendritic cell separation kit (Miltenyi Biotec, Auburn, CA) and a magnetic cell sorting system (Vario MACS: Miltenyi Biotec, Auburn, CA). The results of measuring the number (TLR agonist) of the isolated plasmacytoid dendritic cells are shown in FIG. 17. In this connection, in order to check the effect from the treatment using the toll-like receptor agonist, the non-treatment case using the toll-like receptor agonist during the differentiation of the immature dendritic cells is shown in Comparative Example (non).

As shown in FIG. 17, when the toll-like receptor agonist is applied to the immature dendritic cells during differentiation of the immature dendritic cells (TLR agonist), the number of plasmacytoid dendritic cells as differentiated in the induced manner was significantly increased compared with that of the non-treatment (Non).

Thus, when the treatment using the toll-like receptor agonists is carried out during differentiation of the immature dendritic cells, a large amount of tolerogenic plasmacytoid dendritic cells could be produced.

From the foregoing, it will be appreciated that various embodiments of the present disclosure have been described herein for purposes of illustration, and that various modifications may be made without departing from the scope and spirit of the present disclosure. Accordingly, the various embodiments disclosed herein are not intended to be limiting, with the true scope and spirit being indicated by the following claims.

## Claims

1. A method for producing a tolerogenic plasmacytoid dendritic cell (pDC), the method comprising treating an immature dendritic cell using a toll-like receptor agonist.

2. The method of claim 1, wherein the toll-like receptor agonist is applied to the immature dendritic cell before differentiation initiation of or during the differentiation of the immature dendritic cell, thereby to induce the differentiation of the immature dendritic cell into the tolerogenic plasmacytoid dendritic cell.

3. The method of claim 1, wherein the toll-like receptor agonist is applied to the immature dendritic cell in duration from a start time of differentiation of the immature dendritic cell to a completion time of the differentiation of the immature dendritic cell into the tolerogenic plasmacytoid dendritic cell.

4. The method of claim 2 or 3, wherein the differentiation of the immature dendritic cell into the tolerogenic plasmacytoid dendritic cell is performed using a differentiation-inducing factor.

5. The method of claim 4, wherein the differentiation-inducing factor includes FMS-like tyrosine kinase 3 (Flt3L).

6. The method of claim 4, wherein the differentiation-inducing factor is applied to the immature dendritic cell at one or more times.

7. The method of claim 1, wherein the toll-like receptor agonist and the differentiation-inducing factor are concurrently applied to the immune dendritic cell.

8. The method of claim 1, wherein the toll-like receptor agonist is applied to the immature dendritic cell at one or more times.

9. The method of claim 1, wherein the toll-like receptor agonist affects MyD88 (Myeloid differentiation primary response gene 88) signal.

10. The method of claim 1, wherein the toll-like receptor agonist includes at least one selected from a group consisting of a TLR2 agonist, a TLR4 agonist, a TLR5 agonist, a TLR7 agonist, a TLR8 agonist, a TLR9 agonist, a TLR11 agonist, a TLR12 agonist and a TLR13 agonist.

11. The method of claim 10, wherein the toll-like receptor agonist further includes at least one of TLR1 and TLR6 agonists.

12. The method of claim 2, wherein the method further includes additionally applying the toll-like receptor agonist to the differentiated tolerogenic plasmacytoid dendritic cell, thereby to activate immune tolerance.

13. The method of claim 12, wherein the toll-like receptor agonist used to activate the immune tolerance is a TLR9 agonist.

14. A tolerogenic plasmacytoid dendritic cell induced by treating the immature dendritic cell using a toll-like receptor agonist.

15. The tolerogenic plasmacytoid dendritic cell of claim 14, wherein the tolerogenic plasmacytoid dendritic cell is differentiated in a manner induced by treatment of the immature dendritic cell using the toll-like receptor agonist prior to differentiation initiation of or during the differentiation of the immature dendritic cell.

16. The tolerogenic plasmacytoid dendritic cell of claim 14, wherein the toll-like receptor agonist includes at least one selected from a group consisting of a TLR2 agonist, a TLR4 agonist, a TLR5 agonist, a TLR7 agonist, a TLR8 agonist, a TLR9 agonist, a TLR11 agonist, a TLR12 agonist and a TLR13 agonist.

17. The tolerogenic plasmacytoid dendritic cell of claim 16, wherein the toll-like receptor agonist further includes at least one of TLR1 and TLR6 agonists.

18. A cell therapeutic agent comprising the tolerogenic plasmacytoid dendritic cell of any one of claims 14 to 17.

19. The cell therapeutic agent of claim 18, wherein the cell therapeutic agent induces differentiation into regulatory T cells.

20. The cell therapeutic agent of claim 18, wherein the cell therapeutic agent inhibits activity of effector T cells.
